# EUROPEAN PATENT APPLICATION

(11) **EP 2 335 564 A2**
(43) Date of publication of application: **22.06.2011**
(21) Application number: 10192105.4
(22) Date of filing: 22.11.2010
(51) Int. Cl.: A61B 5/00

(54) **Medical Diagnosis Apparatus and Method of Operating the Same**

(30) Priority: 17.12.2009 KR 20090126080
(71) Applicant: Medison Co., Ltd., Kangwon-do 250-875 (KR)
(72) Inventor: Lee, Su Myeong, Gyeonggi-do (KR); Lee, Yong Ho, Seoul (KR)
(74) Representative: Schmid, Wolfgang

(57) **Abstract**

The present disclosure relates to a medical diagnosis apparatus and a method of operating the same. The medical diagnosis apparatus performs measurement based on a preset diagnosis system environment in response to a measurement start instruction input from a user, and only if the apparatus receives an instruction for changing the diagnosis system environment, the apparatus performs measurement based on a changed diagnosis system environment in response to the instruction.

In the apparatus and method, a workflow is arranged to perform measurement effectively which is a main purpose of the medical diagnosis apparatus, thereby reducing time for measurement or diagnosis and user's operation failure and providing convenience in operation.

## Description

### BACKGROUND OF THE INVENTION

### 1. Field of the Invention

The present invention relates to a medical diagnosis apparatus and a method of operating the same and, more particularly, to a medical diagnosis apparatus and a method of operating the same, which has a workflow arranged to perform measurement effectively which is a main purpose of the medical diagnosis apparatus, thereby reducing time for measurement or diagnosis and user's operation failure and providing convenience in operation.

### 2. Description of the Related Art

Generally, a medical diagnosis apparatus such as an ultrasonic diagnostic apparatus has many functions to achieve desired measurement and diagnosis. The functions of the medical diagnosis apparatus may be independently or cooperatively performed. Herein, the performance of a series of the functions is referred to as a workflow.

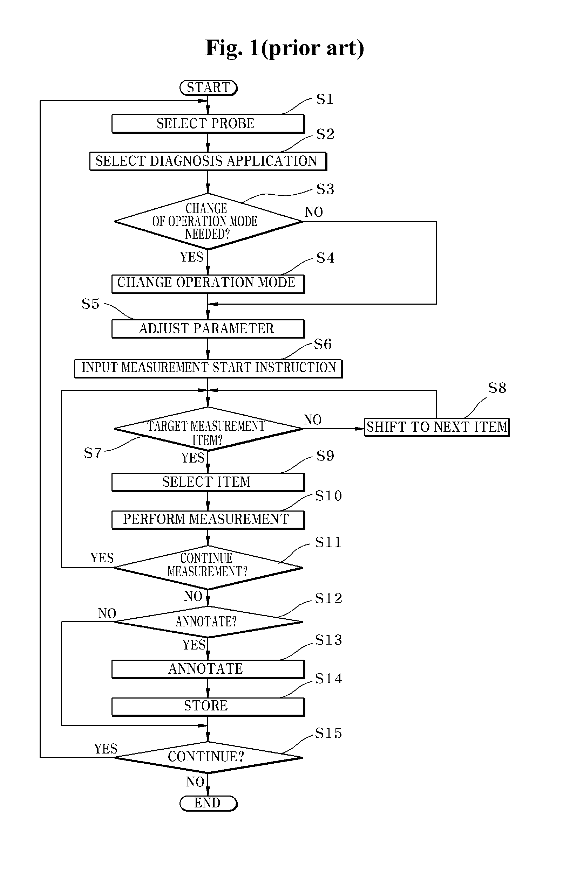

A conventional medical diagnosis apparatus unnecessarily repeats routine operations for measurement, as shown in Fig. 1, thereby causing time consumption and inconvenience in operation. This is described in more detail with reference to Fig. 1. Fig. 1 is a flowchart of a method of operating a conventional medical diagnosis apparatus, for example, an ultrasonic diagnostic apparatus.

Referring to Fig. 1, for the conventional ultrasonic diagnostic apparatus, a user first selects a probe in S1 and then selects a diagnosis application in S2. Here, the term "diagnosis application" means a certain medical department or field, such as obstetrics, genecology, and the like.

Then, it is confirmed in S3 whether a change of an operation mode is needed. If the change of the operation mode is needed, the operation mode is changed in response to a user's selection in S4 and various kinds of parameters related to a diagnosis image are also adjusted in S5. Then, the apparatus receives a measurement start instruction from the user in S6. If it is confirmed in S3 that the change of the operation mode is not needed, the process directly proceeds to S5. Here, the term "operation mode" means a variety of modes related to a diagnosis image of the medical diagnosis apparatus, for example, a two-dimensional (2D) display mode, three-dimensional (3D) display mode, Doppler mode, color mode, and the like. Further, the parameters may include a variety of parameters related to the diagnosis image of the medical diagnosis apparatus, for example, a scale, zoom, focus, time gain compensation (TGC), gain, and the like.

Next, it is confirmed in S7 whether a measurement item to be currently performed is a target item. If the measurement item to be currently performed is the target item, the apparatus receives an item selection signal input from the user in S9. If it is confirmed in S7 that the measurement item to be currently performed is not the target item, the user shifts to the next item in S8 and performs the confirmation operation again in S7. Here, the term "measurement item" may mean a specific portion of an object to be measured by the medical diagnosis apparatus, or a specific measurement item of the specific portion. For example, when a fetus is subjected to ultrasound diagnosis, the measurement item means a specific portion, such as the head, the legs, the stomach, the womb, or the like, for each group, such as an initial fetus, a general fetus, or the like, or means a specific measurement item of the specific portion, such as a diameter of the head of a fetus.

Next, if the measurement item is selected in S9, the measurement is performed in S10. Then, it is confirmed in S11 whether the user wants to continue the measurement. If the user wants to continue the measurement, the process returns back to the operation in S7 and repeats the above operations. Here, the confirmation of whether the user wants to continue the measurement is to confirm whether the user wants to continue the measurement under diagnosis conditions set in S1∼S5.

Next, if it is confirmed in S11 that the user does not want to continue the measurement, it is confirmed in S12 whether the user wants to annotate a measurement result. If the user wants to annotate the measurement result, the annotation is provided to the result in S13, and the content of annotation is stored in a storage in S14, after the annotation operation is finished. If the user does not want to annotate the result, the process proceeds to S15 described below. Herein, the annotation includes a body marker as well as a general annotation.

Finally, it is confirmed whether the user wants to continue the measurement, and if the answer is yes, the process returns back to S1 and repeats the above operations. If the answer is no, the measurement is finished in S15.

Such a conventional medical diagnosis apparatus has problems as follows.

First, although the main purpose of the medical diagnosis apparatus such as an ultrasonic diagnostic apparatus is to obtain an image of a diagnosis object and a diagnosis result based on measurement of the image, a user has to exert considerable effort to perform a number of operations involved in the measurement. Such effort makes it difficult for the user to concentrate on the actual measurement, thereby deteriorating operation efficiency.

Second, even in the case where the measurement is repetitiously performed according to the same workflow, the conventional apparatus requires a user to inconveniently repeat a series of operations, for example, searching for or moving to a target measurement item and then selecting the target measurement item. Accordingly, there is a problem in that operation efficiency is deteriorated due to an increase in operation depth and operation time. Here, the operation depth means the number or extent of operations that a user must perform to select a desired measurement item.

### SUMMARY OF THE INVENTION

The present invention is conceived to solve the problems of the related, and an aspect of the present invention is to provide a medical diagnosis apparatus and a method of operating the same, which may prevent an increase in operation time due to unnecessary repetition of routine operations frequently performed in use of the medical diagnosis apparatus, thereby providing convenience in operation.

In accordance with an aspect, the present invention provides a method of operating a medical diagnosis apparatus, wherein the medical diagnosis apparatus performs measurement based on a preset diagnosis system environment in response to a measurement start instruction input from a user, and wherein only if the apparatus receives an instruction for changing the diagnosis system environment, the apparatus performs measurement based on a changed diagnosis system environment in response to the instruction.

The method may include: 1) receiving the measurement start instruction; 2) confirming whether a change of the preset diagnosis system environment is needed; and 3) performing the measurement, wherein the measurement is performed by reflecting the change in diagnosis system environment only upon confirming that the change of the preset diagnosis system environment is needed.

The diagnosis system environment may include information about at least one of a probe, a diagnosis application, an operation mode, and at least one parameter.

The step 2) of confirming may include: a) confirming whether a change of a diagnosis application is needed; and b) confirming whether a change of an operation mode is needed, wherein the step b) is performed after changing the diagnosis application only upon confirming that the change of the diagnosis application is needed.

The step 2) of confirming may include: a) confirming whether a change of an operation mode is needed; and b) confirming whether a change of a diagnosis application is needed, wherein the step b) is performed after changing the operation mode only upon confirming that the change of the operation mode is needed.

The step 2) of confirming may further include optionally: c) confirming whether a change of a probe is needed, before the step a), after the step a), or after the step b), wherein the probe is changed to proceed to the next step, only upon confirming that the change of the probe is needed.

The step 2) of confirming may further include optionally: d) confirming whether a change of a parameter is needed, before the step a), after the step a), or after the step b), wherein the parameter is changed to proceed to the next step, only upon confirming that the change of the parameter is needed.

The step 2) of confirming may further include: c) confirming whether a change of a probe is needed; and d) confirming whether a change of a parameter is needed, wherein the steps c) and d) may be performed at any selective time point.

The method may further include : confirming whether a user wants to continue to operate the medical diagnosis apparatus after the step 3) of performing the measurement; and returning to the step 2) of confirming whether a change of the preset diagnosis system environment is needed, upon confirming that the user wants to continue to operate the medical diagnosis apparatus.

The method may further include annotating a measurement result in response to a selection of the user between the step 3) of performing measurement and the step 4) of confirming whether the user wants to continue to operate the medical diagnosis apparatus.

The at least one parameter may include at least one of a scale, zoom, focus, time gain compensation (TGC), and gain.

The preset diagnosis system environment may be set by a user using an editing function.

In accordance with another aspect, the present invention provides a medical diagnosis apparatus including: an input unit receiving an input from a user; a storage storing information about a preset diagnosis system environment; a controller controlling the apparatus to perform measurement based on the preset diagnosis system environment; and an output unit, wherein the controller controls the medical diagnosis apparatus to perform the measurement based on the preset diagnosis system environment in response to a measurement start instruction input from the user, and wherein only if the apparatus receives an instruction for changing the diagnosis system environment, the controller allows the apparatus to perform measurement based on a changed diagnosis system environment in response to the instruction.

The controller may confirm whether a change of the preset diagnosis system environment is needed, in response to the measurement start instruction input, and control the apparatus to perform the measurement if the change of the preset diagnosis system condition is not needed, while allowing the apparatus to perform the measurement by reflecting the change in diagnosis system environment only if the change of the preset diagnosis system environment is needed.

The diagnosis system environment may include information about at least one of a probe, a diagnosis application, an operation mode, and at least one parameter.

When changing the preset diagnosis system environment, the apparatus may change at least one of the probe, the diagnosis application, the operation mode, and the at least one parameter.

The apparatus may further include an editing unit for editing or changing the preset diagnosis system environment.

### BRIEF DESCRIPTION OF THE DRAWINGS

The above and other aspects, features and advantages of the invention will become apparent from the following description of embodiments given in conjunction with the accompanying drawings, in which:
- Fig. 1: is a flowchart of a method of operating a conventional medical diagnosis apparatus;
- Fig. 2: is a block diagram of a medical diagnosis apparatus according to one embodiment of the present invention; and
- Fig. 3: is a flowchart of a method of operating a medical diagnosis apparatus according to one embodiment of the present invention.

### DETAILED DESCRIPTION OF THE EMBODIMENT

Exemplary embodiments of the invention will now be described in detail with reference to the accompanying drawings. It should be noted that these embodiments are provided for illustration only and do not limit the scope of the present invention.

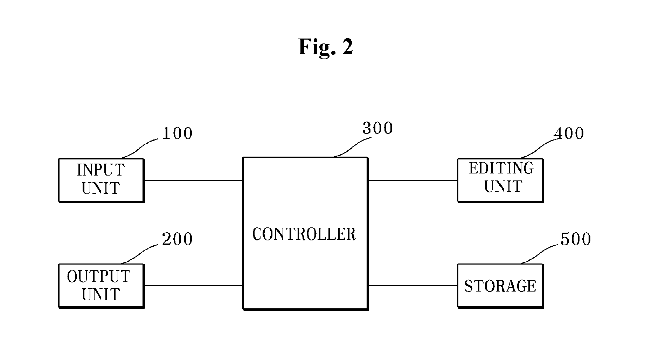

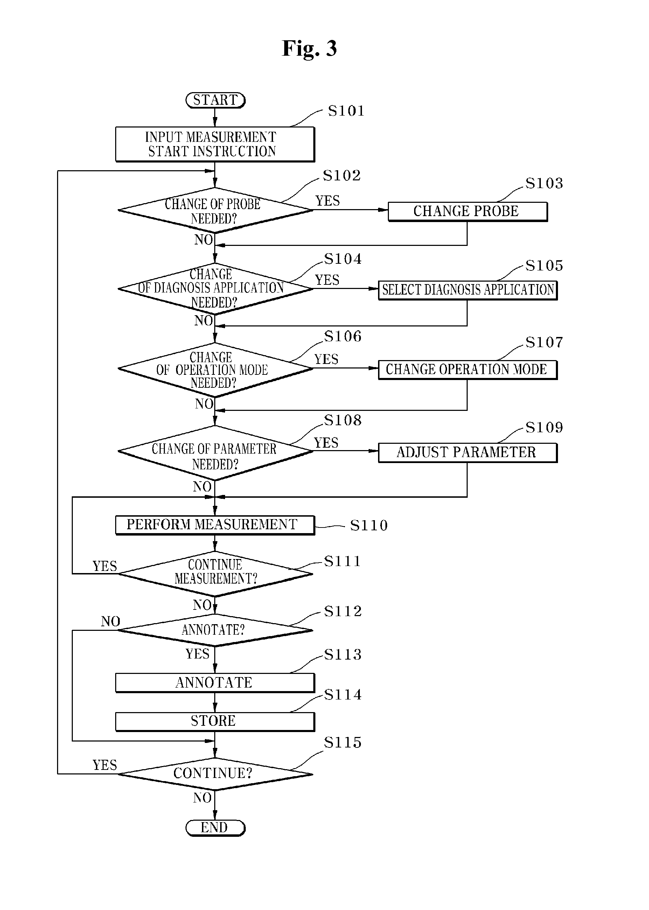

Fig. 2 is a block diagram of a medical diagnosis apparatus according to one embodiment of the present invention, and Fig. 3 is a flowchart of a method of operating a medical diagnosis apparatus according to one embodiment of the present invention.

Referring to Fig. 2, the medical diagnosis apparatus of this embodiment includes an input unit 100 receiving an input from a user; a storage 500 storing information about a preset diagnosis system environment; a controller 300 controlling the apparatus to perform measurement based on the preset diagnosis system environment; an output unit 200; and an editing unit 400 allowing the user to edit or change the preset diagnosis system environment.

In the medical diagnosis apparatus of the embodiment, information of a diagnosis system environment is preset and stored in the storage 500. Here, the information of the diagnosis system environment may be an initial preset value originally stored in the medical diagnostic apparatus. Further, the information of the diagnosis system environment may be newly set by a user using the editing unit 400 or may be reset by editing or changing a certain preset value using the editing unit 400. The diagnosis system environment may include a diagnosis application, an operation mode, at least one measurement parameter, and other preset conditions, which may be set to perform measurement in the medical diagnosis apparatus. In particular, for an ultrasonic diagnostic apparatus, the diagnosis system environment may include information about probes. The diagnosis application, operation mode and measurement parameters are the same as those disclosed in the background of the invention.

A method of operating the medical diagnosis apparatus according to one embodiment is described with reference to Fig. 3.

Referring to Fig. 3, in the method of the embodiment, first, the medical diagnosis apparatus receives a measurement start instruction input from a user in S101, and it is confirmed in S102 whether a change of a probe is needed. That is, in the method according to the embodiment, the measurement start instruction is first input in order to achieve satisfactory measurement, that is, a main purpose of the medical diagnosis apparatus.

If it is confirmed in S102 that the change of the probe is not needed, the process proceeds to S104. Only if the change of the probe is needed, the probe is changed to a newly selected probe in S103. Herein, the operations in S102 and S103 related to change or selection of the probe may also be applied to an ultrasonic diagnostic apparatus and may be omitted depending on the kind of medical diagnosis apparatus.

Then, it is confirmed in S104 whether a change of a diagnosis application is needed. If it is confirmed in S104 that the change of the diagnosis application is not needed, the process proceeds to S106. Only if the change of the diagnosis application is needed, the diagnosis application is changed to a newly selected diagnosis application in S105.

Then, it is confirmed in S106 whether a change of an operation mode is needed. If it is confirmed in S106 that the change of the operation mode is not needed, the process proceeds to S108. Only if the change of the operation mode is needed, the operation mode is changed to a newly selected operation mode in S107.

Then, it is confirmed in S108 whether a change of a parameter is needed. If it is confirmed that the change of the parameter is not needed, the process proceeds to the next operation to perform measurement in S110. Only if the change of the parameter is needed, parameters related to various kinds of diagnosis images are modified or adjusted in S109 and the measurement is performed in S110.

As such, in this embodiment, the medical diagnosis apparatus confirms, through the operations in S102, S104, S106 and S108, whether a change of the preset diagnosis system environment including at least one of the probe, diagnosis application, operation mode and at least one parameter is needed. The medical diagnosis apparatus directly performs the measurement if it is confirmed that the change of the preset diagnosis system environment is not needed, so that the number of unnecessary operations is minimized in performing the measurement, thereby reducing required operating time and operating frequency of the user required for an actual measurement or diagnosis and providing convenience in operation.

Next, it is confirmed in S111 whether a user wants to continue the measurement. If the answer is yes, the process returns back to the operation in S110 to perform the measurement, and if the answer is no, the process proceeds to S112. Here, the confirmation of whether the user wants to continue the measurement is to confirm whether the user wants to continue the measurement under the overall diagnosis system environment preset in the current stage. When the process returns back to the operation in S110 to continue the measurement, the method may further include searching for a specific measurement item to allow a user to select the specific measurement item as a desired one, if the user wants to change a current measurement item to the specific measurement item to be performed by the diagnostic apparatus.

If it is confirmed in S111 that the user does not want to continue the measurement, it is confirmed in S112 whether the user wants to annotate a measurement result. If it is confirmed that the user wants to annotate the result, the annotation is provided to the result in S113 and the content of annotation is stored in the storage 500 after the annotation operation is finished, in S114. If it is confirmed that the user does not want to annotate the result, the process proceeds to step S115 described below.

Next, if it is confirmed that the user wants to continue the measurement, the process returns back to S102 and repeats the above operations. If the user does not want to continue the measurement, the measurement is finished in S115. Here, according to another embodiment, the process may return back from the operation in 5115 to any one of the operations in S104, S106 6 and S108 8 instead of returning back to the operation in S102.

According to another embodiment, the operations in S102, S104 S106 and S108 may be performed in a different sequence from the embodiment shown in Fig. 3. In other words, for example, the operations in S106 and S108 may be performed before the operations in S102 and S104. Further, at least one of the operations in S102, S104 S106 and S108 may be selectively applied.

As such, in the medical diagnosis apparatus and method of operating the same according to the embodiments, a workflow is arranged to perform the measurement effectively which is a main purpose of the medical diagnosis apparatus, such that general operations except for the measurement may be automatically set and performed based on a preset diagnosis system environment and may be changed to reflect a change or modification of the diagnosis system environment if needed, thereby reducing time for measurement or diagnosis and operation failure and providing convenience in operation.

Although some embodiments have been provided to illustrate the invention in conjunction with the drawings, it is apparent to those skilled in the art that the embodiments are given by way of illustration only, and that various modifications and equivalent embodiments can be made without departing from the spirit and scope of the invention. Therefore, the scope of the invention should be limited only by the accompanying claims and equivalents thereof.

## Claims

1. A method of operating a medical diagnosis apparatus, wherein the medical diagnosis apparatus performs measurement based on a preset diagnosis system environment in response to a measurement start instruction input from a user, and wherein only if the apparatus receives an instruction for changing the diagnosis system environment, the apparatus performs measurement based on a changed diagnosis system environment in response to the instruction.

2. The method of claim 1, comprising:
1) receiving the measurement start instruction;
2) confirming whether a change of the preset diagnosis system environment is needed; and
3) performing the measurement,
wherein the measurement is performed by reflecting the change in diagnosis system environment only upon confirming that the change of the preset diagnosis system environment is needed.

3. The method of claim 2, wherein the diagnosis system environment comprises information about at least one of a probe, a diagnosis application, an operation mode, and at least one parameter.

4. The method of claim 3, wherein the step 2) of confirming whether the change of the preset diagnosis system environment is needed comprises:
a) confirming whether a change of a diagnosis application is needed; and
b) confirming whether a change of an operation mode is needed,
the step b) being performed after changing the diagnosis application only upon confirming that the change of the diagnosis application is needed.

5. The method of claim 3, wherein the step 2) of confirming whether the change of the preset diagnosis system environment is needed comprises:
a) confirming whether a change of an operation mode is needed; and
b) confirming whether a change of a diagnosis application is needed,
the step b) being performed after changing the operation mode only upon confirming that the change of the operation mode is needed.

6. The method of claim 4, wherein the step 2) of confirming whether the change of the preset diagnosis system environment is needed further comprises optionally:
c) confirming whether a change of a probe is needed, before the step a), after the step a), or after the step b),
the probe being changed to proceed to the next step only upon confirming that the change of the probe is needed.

7. The method of claim 4, wherein the step 2) of confirming whether the change of the preset diagnosis system environment is needed further comprises optionally:
d) confirming whether a change of a parameter is needed, before the step a), after the step a), or after the step b),
the parameter being changed to proceed to the next step only upon confirming that the change of the parameter is needed.

8. The method of claim 4, wherein the step 2) of confirming whether the change of the preset diagnosis system environment is needed further comprises:
c) confirming whether a change of a probe is needed; and
d) confirming whether a change of a parameter is needed,
wherein the steps c) and d) are performed at any selective time point.

9. The method of claim 4, further comprising:
confirming whether a user wants to continue to operate the medical diagnosis apparatus after the step 3) of performing the measurement; and
returning to the step 2) of confirming whether a change of the preset diagnosis system environment is needed, upon confirming that the user wants to continue to operate the medical diagnosis apparatus.

10. The method of claim 9, further comprising:
annotating a measurement result in response to a selection of the user between the step 3) of performing measurement and the step 4) of confirming whether the user wants to continue to operate the medical diagnosis apparatus.

11. The method of claim 3, wherein the at least one parameter comprises at least one of a scale, zoom, focus, time gain compensation (TGC), and gain.

12. A medical diagnosis apparatus comprising:
an input unit receiving an input from a user;
a storage storing information about a preset diagnosis system environment;
a controller controlling the apparatus to perform measurement based on the preset diagnosis system environment; and
an output unit,
wherein the controller controls the medical diagnosis apparatus to perform the measurement based on the preset diagnosis system environment in response to a measurement start instruction input from the user, and
wherein only if the apparatus receives an instruction for changing the diagnosis system environment, the controller allows the apparatus to perform measurement based on a changed diagnosis system environment in response to the instruction.

13. The medical diagnosis apparatus of claim 12, wherein the controller confirms whether a change of the preset diagnosis system environment is needed, in response to the measurement start instruction input, and controls the apparatus to perform the measurement if the change of the preset diagnosis system condition is not needed, while allowing the apparatus to perform the measurement by reflecting the change in diagnosis system environment only if the change of the preset diagnosis system environment is needed.

14. The medical diagnosis apparatus of claim 13, wherein the diagnosis system environment comprises information about at least one of a probe, a diagnosis application, an operation mode, and at least one parameter.

15. The medical diagnosis apparatus of claim 14, wherein, when changing the preset diagnosis system environment, the apparatus changes at least one of the probe, the diagnosis application, the operation mode, and the at least one parameter.
